# EUROPEAN PATENT APPLICATION

(11) **EP 3 076 179 A1**
(43) Date of publication of application: **05.10.2016**
(21) Application number: 15161647.1
(22) Date of filing: 30.03.2015
(51) Int. Cl.: G01N 33/574

(54) **DIAGNOSIS AND TREATMENT OF LOW GRADE GLIOMAS**

(71) Applicant: Deutsches Krebsforschungszentrum Stiftung des Öffentlichen Rechts, 69120 Heidelberg (DE); Ludwig-Maximilians-Universität München, 80539 München (DE)
(72) Inventor: The designation of the inventor has not yet been filed
(74) Representative: Heubeck, Christian

(57) **Abstract**

The present invention relates to methods for diagnosing or treating an individual with low grade glioma at risk to undergo malignant progression.

## Description

### FIELD OF THE INVENTION

The present invention relates to methods for diagnosing and/or treating an individual with low grade glioma at risk to undergo malignant progression.

### BACKGROUND

Gliomas are by far the most common primary brain tumors in adults and account for more than 50% of all brain tumors. The main types of neuroepithelial tumors are ependymomas, astrocytomas, and oligodendrogliomas, although there also exist mixed cellular forms, such as oligoastrocytomas. The World Health Organization classifies astrocytomas according to their degree of anaplasia into grade II (diffuse astrocytoma), grade III (anaplastic astrocytoma) and grade IV (glioblastoma: GBM)1.In adults, the most common tumors are high-grade neoplasms derived from astrocytes or oligodendrocytes than are diagnosed as such (primary GBM) or develop from a more benign astrocytoma (secondary GBM). Generically, high grade gliomas are highly vascular tumors and have a tendency to infiltrate tissues, create necrosis and hypoxia, and destroy the blood-brain barrier where the tumor is located. "Low grade" gliomas are typically well-differentiated, slower growing, biologically less aggressive, and portend a better prognosis for the patient; while the "high grade" gliomas, are undifferentiated or anaplastic, fast growing and can invade adjacent tissues, and carry a worse prognosis.

The current treatment of malignant gliomas (grade III and IV) involves surgery, followed by irradiation and chemotherapy. As low grade gliomas (LGG) are considered as benign tumors which are not immediately life threatening for patients, as e.g. high-grade gliomas (GBM)¹, clinical treatments for LGG often include wait-and-see strategies² and rarely comprise aggressive multimodal treatments as usually applied to GBM patients³. However, upon molecular imaging LGG are often "under-rated" and are e.g. diagnosed as grade II tumors although anaplastic foci within a tumor may also allow to classify these gliomas as grade III tumors⁴. Hence, grade II gliomas can be "under-treated" and may undergo accelerated malignant progression. In order to provide an up-front diagnosis that indicates LGG patients which are at risk to suffer from malignant progression, an imaging procedure was developed, that predicts malignant progression with high confidence⁴. It was found, that FET-PET, which provides data on tumor cell metabolism, shows different kinetics throughout dynamic measurements (over a time-course of 40min) for benign and anaplastic LGGs5. FET-PET signals which have static (constant) kinetics indicate benign tumors and FET-PET signals with dynamic kinetics give a strong indication for LGGs which are at risk to progress into higher grade tumors.

FET-PET can thus substantially improve early diagnosis of LGG patients which need advanced therapies. However, the drawbacks of this approach are that dynamic FET-PET is not commonly established in clinical settings and does not give any further indications for targeted therapy.

Thus, there was a need in the art for a diagnostic method that can more easily be established in clinical settings and yet provides a reliable prognosis, whether a low grade glioma is at risk to undergo malignant progression.

In the present invention it was found, that CD95L (FAS-ligand, FAS-LG) can serve as a new marker for LGGs with anaplastic foci and thus a high risk to undergo malignant progression.

### DESCRIPTION

The present invention provides a method for the diagnosis of low grade glioma (LGG) at risk to undergo malignant progression, comprising
(i) determining the expression level of CD95L in a sample from a patient affected with LGG and
(ii) classifying the LGG as being at risk to undergo malignant progression dependent on the level of CD95L expression.

Another aspect of the invention is a method for the prognosis of malignant progression of LGG, comprising
(i) determining the expression level of CD95L in a sample from a patient affected with LGG and
(ii) classifying the LGG as being at risk to undergo malignant progression dependent on the level of CD95L expression.

The sample employed in the diagnostic or prognostic method as described herein can be a freshly obtained sample or an archived tumor tissue, for example a biopsy or surgery material embedded in paraffin, which has been obtained in an earlier stage of the disease.

In the present invention, expression of CD95L can be determined by any known suitable method. The expression of CD95L can be determined on protein level or nucleic acid level. Examples of suitable methods for determining CD95L include histological, histochemical or/and immunohistochemical methods. For example, the CD95L protein or CD95L mRNA can be determined. Suitable methods for detection of CD95L are described e.g. in PCT/EP2014/058746. CD95L mRNA might be determined by quantitative real-time PCR.

CD95L expression in cells can particularly be determined by contacting the sample with an agent specifically binding to CD95L. Such an agent may bind to unbounded ("free") CD95L or CD95L bound in the CD95L/CD95-complex. For example, antibodies specifically binding to CD95L can be used. Suitable antibodies can be prepared by known methods. Further suitable agents specifically binding to CD95L include an extracellular receptor domain of CD95, or a functional fragment thereof, for example in a fusion polypeptide further comprising an Fc domain, or a functional fragment thereof. An example of a suitable fusion polypeptide is APG101, as described herein below. The determination of CD95L can be assisted by suitable labeling, staining methods and/or molecular imaging methods well known in the art. A preferred embodiment of staining CD95L is staining and counterstaining by hematoxilin.

An LGG can be regarded as CD95L positive and thus at risk to undergo malignant progression, if at least 20%, of the cells in a sample express CD95L. The number of CD95L positive cells can be determined e.g. by counting the cells in a microscopic section.

CD95L expression is considered to be absent (CD95L negative) if essentially no cells expressing CD95L can be detected in the tissue sample, or if the sample is a sample which does not fulfill the criteria defined herein for a CD95L positive sample (non-positive sample). In a CD95L negative sample, the number of tumor cells expressing CD95L can be below the threshold defined herein for CD95L positive samples, for example below 3% of tumor cells. If CD95L expression is absent, the LGG is classified as benign, and thus to have a low risk to undergo malignant progression.

An LGG can also be regarded as CD95L positive and thus at risk to undergo malignant progression, if CD95L can be detected on at least 20% of the area of tumor tissue in a tissue section. This value is termed herein as "% CD95L positive area of tumor tissue". Non-tumor tissue is excluded in this analysis. A tissue section can be prepared by known methods. CD95L expression can be considered to be absent (CD95L negative) if essentially no CD95L can be detected in the tissue sample, or if the value of % CD95L positive area of tumor tissue is below the threshold defined for a CD95 positive sample, for example below 3% of tumor area. If CD95L expression is absent, the LGG is classified as benign, and thus to have a low risk to undergo malignant progression.

CD95L expression (e.g. in terms of cell number or surface in a tissue section) can be determined, for example by methods based upon automatized analysis of tissue sections.

The patient for whom a diagnosis, prognosis or treatment shall be provided according to the invention can be a patient with first or second relapse or progression of LGG. The LGG may be a WHO Grade I or II glioma, in particular a WHO Grade II glioma.

According to a preferred embodiment of the invention, the expression of CD95L in the sample is determined by contacting the sample with an inhibitor of CD95L. Exemplary inhibitors of CD95L are antagonistic anti-CD95L antibodies as disclosed in EP-A-0 842 948, WO 96/29350, WO 95/13293. Also suitable are chimeric or humanized antibodies obtained therefrom, cf. e.g. WO 98/10070.

Further suitable are soluble CD95 receptor molecules, e.g. a soluble CD95 receptor molecule without transmembrane domain as described in EP-A-0 595 659 and EP-A-0 965 637 or CD95R peptides as described in WO 99/65935, which are herein incorporated by reference.

Further preferred inhibitors are multimeric CD95R fusion polypeptides comprising the CD95R extracellular domain or a fragment thereof and a multimerization domain, particularly a trimerization domain, e.g. bacteriophage T4 or RB69 foldon fusion polypeptides as described in WO 2008/025516, which is herein incorporated by reference. The Fas ligand inhibitor FLINT or DcR3 or a fragment, e.g. a soluble fragment thereof, for example the extracellular domain optionally fused to a heterologous polypeptide, particularly a Fc immunoglobulin molecule is described in WO 99/14330, WO 99/50413 or Wroblewski et al., Biochem. Pharmacol. 65, 657-667 (2003), which are herein incorporated by reference. FLINT and DcR3 are proteins which are capable of binding the CD95 ligand and LIGHT, another member of the TNF family.

According to an especially preferred embodiment of the invention, the CD95L inhibitor and/or CD95L specifically binding agent comprise at least one extracellular domain of the CD95R molecule (particularly amino acids 1 to 172 (MLG ... SRS) of the mature CD95 sequence according to U.S. Pat. No. 5,891,434) optionally fused to a heterologous polypeptide domain, particularly a Fc immunoglobulin molecule including the hinge region e.g. from the human IgG1 molecule. Particularly preferred fusion proteins comprising an extracellular CD95 domain and a human Fc domain are described in WO 95/27735 and PCT/EP2004/003239, which are herein incorporated by reference.

Particularly preferred CD95L inhibitors and/or agents specifically binding CD95L in terms of the invention comprise
(i) a fusion protein comprising at least one extracellular CD95 domain or a functional fragment thereof and at least one Fc domain or a functional fragment thereof and/or
(ii) an anti-CD95L specific antibody or a CD95L recognizing fragment thereof.

The CD95L inhibitor and/or agents specifically binding CD95L employed in the present invention can comprise a fusion protein comprising at least one extracellular CD95 domain or a functional fragment thereof and at least one Fc domain or a functional fragment thereof. In a particularly preferred embodiment, the CD95L inhibitor and/or agents specifically binding CD95L are or comprise a fusion protein selected from APG101, polypeptides having at least 70% identity to APG101 and functional fragments of APG101.

Fusion proteins comprising the extracellular domain of the death receptor CD95 (also called Apo-1 or Fas) fused to an immunoglobulin Fc domain are described in PCT/EP04/003239, the disclosure of which is included herein by reference. "Fusion protein", as used herein, includes a mixture of fusion protein isoforms, each fusion protein comprising at least an extracellular CD95 domain (APO-1; Fas) or a functional fragment thereof and at least a second domain being an Fc domain or a functional fragment thereof distributing within a pl range of about 4.0 to about 8.5. Accordingly, the extracellular CD95 domain as used herein may be also called "first domain", while the Fc domain may be called "second domain".

The first domain protein is an extracellular CD95 domain, preferably a mammalian extracellular domain, in particular a human protein, i.e. a human extracellular CD95 domain. The first domain, i.e. the extracellular CD95 domain, of the fusion protein preferably comprises the amino acid sequence up to amino acid 170, 171, 172 or 173 of human CD95 (SEQ ID NO. 1). A signal peptide (e.g. position 1-25 of SEQ ID NO: 1) may be present or not. Particularly for therapeutic purposes the use of a human protein is preferred.

The fusion protein can comprise one or more first domains which may be the same or different. One first domain, i.e. one extracellular CD95 domain is preferred to be present in the fusion protein.

According to a preferred embodiment, the Fc domain or functional fragment thereof, i.e. the second domain of the fusion protein according to the invention, comprises the CH2 and/or CH3 domain, and optionally at least a part of the hinge region, or a modified immunoglobulin domain derived therefrom. The immunoglobulin domain may be an IgG, IgM, IgD, or IgE immunoglobulin domain or a modified immunoglobulin domain derived, therefrom. Preferably, the second domain comprises at least a portion of a constant IgG immunoglobulin domain. The IgG immunoglobulin domain may be selected from IgG1, IgG2, IgG3 or IgG4 domains or from modified domains therefrom. Preferably, the second domain is a human Fc domain, such as a IgG Fc domain, e.g. a human IgG1 Fc domain.

The fusion protein can comprise one or more second domains which may be the same or different. One second domain, i.e. one Fc domain is preferred to be present in the fusion protein.

Further, both the first and second domains are preferably from the same species.

The first domain, i.e. the extracellular CD95 domain or the functional fragment thereof may be located at the N- or C-terminus. The second domain, i.e. the Fc domain or functional fragment may also be located at the C- or N-terminus of the fusion protein. However, the extracellular CD95 domain at the N-terminus of the fusion protein is preferred.

According to a further preferred embodiment, the fusion protein is APG101 (CD95-Fc, position 26-400 in SEQ ID NO: 1). As defined by SEQ ID NO: 1 APG101 can be a fusion protein comprising a human extracellular CD95 domain (amino acids 26-172) and a human IgG1 Fc domain (amino acids 172-400), further optionally comprising an N-terminal signal sequence (e.g. amino acids 1-25 of SEQ ID NO: 1). The presence of the signal peptide indicates the immature form of APG101. During maturation, the signal peptide is cleaved off. According to an especially preferred embodiment the signal sequence is cleaved off. APG101 with the signal sequence being cleaved off is also comprised by the term "unmodified APG101".

In a further embodiment the fusion protein is a polypeptide having at least 70% identity, more preferably 75% identity, 80% identity, 85% identity, 90% identity, 95% identity, 96% identity, 97% identity, 98% identity, 99% identity with APG101. According to the present application the term "identity" relates to the extent to which two amino acid sequences being compared are invariant, in other words share the same amino acids in the same position.

The term "APG101" includes a fusion protein of position 26-400 of SEQ ID NO: 1, with and without a signal peptide. The term "APG101" also includes fusion proteins containing N-terminally truncated forms of the CD95 extracellular domain.

In another preferred embodiment the fusion protein according to the invention is a functional fragment of APG101. As used herein, the term "fragment" generally designates a "functional fragment", i.e. a fragment or portion of a wild-type or full-length protein which has essentially the same biological activity and/or properties as the corresponding wild-type or full-length protein has.

A person skilled in the art is aware of methods to design and produce fusion proteins according to the present invention. The mixture of fusion protein isoforms, in particular APG101 isoforms, however, can be obtained by a method described, e.g., in PCT/EP04/03239, the disclosure of which is included herein by reference. According to a preferred embodiment designing a fusion protein for the use of the present invention comprises a selection of the terminal amino acid(s) of the first domain and of the second domain in order to create at least one amino acid overlap between both domains. The overlap between the first and the second domain or between the two first domains has a length of preferably 1, 2 or 3 amino acids. More preferably, the overlap has a length of one amino acid. Examples for overlapping amino acids are S, E, K, H, T, P, and D.

As indicated above, "fusion protein", as used herein, includes a mixture of isoforms. The term "isoform" as used herein designates different forms of the same protein, such as different forms of APG101, in particular APG101 without signal sequence. Such isoforms can differ, for example, by protein length, by amino acid, i.e. substitution and/or deletion, and/or post-translational modification when compared to the corresponding unmodified protein, i.e. the protein which is translated and expressed from a given coding sequence without any modification. Different isoforms can be distinguished, for example, by electrophoresis, such as SDS-electrophoresis, and/or isoelectric focussing which is preferred according to the present invention.

Isoforms differing in protein length can be, for example, N- terminally and/or C-terminally extended and/or shortened when compared with the corresponding unmodified protein. For example, a mixture of APG101 isoforms according to the invention can comprise APG101 in unmodified form as well as N- terminally and/or C-terminally extended and/or shortened variants thereof. Thus, according to a preferred embodiment, the mixture according to the invention comprises N-terminally and/or C-terminally shortened variants of APG101. In particular preferred is a mixture of fusion protein isoforms comprising N-terminally shortened fusion proteins. Such N-terminally shortened fusion proteins may comprise -1, -2, -3, -4, -5, -6, -7, -8, -9, -10, -11, -12, -13, - 14, -15, -16, -17, -18, -19, -20, -21, -22, -23, -24, -25, -26, -27, -28, -29, -30, - 35, -40, -45 and/or -50 N-terminally shortened variants of unmodified APG101. Particularly preferred are -17, -21 and/or -26 N-terminally shortened variants. The numbering refers to the APG101 protein including signal sequence according to SEQ ID NO: 1. In other words, the shortened fusion proteins can comprise a sequence SEQ ID NO: 1 N-terminally truncated by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45 and/or 50 amino acids. Preferred shortened fusion proteins have SEQ ID NO: 1 N-terminally truncated by 16, 20, or 25 amino acids.

According to the invention, the mixture of fusion protein isoforms distributes within a pl range of about 4.0 to about 8.5. In a further embodiment the pl range of the mixture of fusion protein isoforms comprised by the composition according to the invention is about 4.5 to about 7.8, more preferably about 5.0 to about 7.5.

The isoelectric point (pl) is defined by the pH-value at which a particular molecule or surface carries no electrical charge. Depending on the pH range of the surrounding medium the amino acids of a protein may carry different positive or negative charges. The sum of all charges of a protein is zero at a specific pH range, its isoelectric point, i.e. the pl value. If a protein molecule in an electric field reaches a point of the medium having this pH value, its electrophoretic mobility diminishes and it remains at this site. A person skilled in the art is familiar with methods for determining the pl value of a given protein, such as isoelectric focusing. The technique is capable of extremely high resolution. Proteins differing by a single charge can be separated and/or fractionated.

An example for a C-terminal shortening of APG101 isoforms is C-terminal Lys-clipping.

As outlined above, isoforms can also differ by amino acid substitution, amino acid deletion and/or addition of amino acids. Such a substitution and/or deletion may comprise one or more amino acids. However, the substitution of a single amino acid is preferred according to this embodiment.

Isoforms according to the invention can also differ with regard to post-translational modification. Post-translational modification according to the present invention may involve, without being limited thereto, the addition of hydrophobic groups, in particular for membrane localisation such as myristoylation, palmitoylation, isoprenylation or glypiation, the addition of cofactors for enhanced enzymatic activity such as lipoyation, the addition of smaller chemical groups such as acylation, formylation, alkylation, methylation, amidation at the C-terminus, amino acid addition, γ-carboxylation, glycosylation, hydroxylation, oxidation, glycilation, biotinylation and/or pegylation.

According to a preferred embodiment the fusion proteins for use according to the present invention are comprised in a composition comprising high amounts of sialic acids. According to the present invention the content of sialic acid is preferably from about 4.0 to 7.0 mol NeuAc/mol APG101, more preferably from 4.5 to 6.0 mol NeuAc/mol APG101 and most preferably about 5.0 mol NeuAc/mol APG101. As used herein, sialic acids refer N- or O-substituted derivatives of neuraminic acid.

According to the present invention, glycosylation designates a reaction in which a carbohydrate is attached to a functional group of a fusion protein, functional fragment thereof as defined herein. In particular, it relates to the addition of a carbohydrate to APG101 or an isoform thereof. The carbohydrate may be added, for example, by N-linkage or O-linkage. N-linked carbohydrates are attached to a nitrogen of asparagine or arginine site chains. O-linked carbohydrates are attached to the hydroxy oxygen of serine, threonine, tyrosine, hydroxylysine or hydroxyproline side chains.

Fucosylation according to the present invention relates to the adding of fucose sugar units to a molecule. With regard to the present invention such an addition of a fucose sugar unit to the fusion protein, and in particular to APG101, represents an especially preferred type of glycosylation.

Beside the first and second domain as defined herein, the fusion proteins for use according to the invention may comprise further domains such as further targeting domains, e.g. single chain antibodies or fragments thereof and/or signal domains. According to a further embodiment, the fusion protein used according to the invention may comprise an N-terminal signal sequence, which allows secretion from a host cell after recombinant expression. The signal sequence may be a signal sequence which is homologous to the first domain of the fusion protein. Alternatively, the signal sequence may also be a heterologous signal sequence. In a different embodiment the fusion protein is free from an additional N-terminal sequence, such as a signal peptide.

The fusion protein as described herein may be an N-terminally blocked fusion protein, which provides a higher stability with regard to N-terminal degradation by proteases, as well as a fusion protein having a free N-terminus, which provides a higher stability with regard to N-terminal degradation by proteases.

Modifications blocking the N-terminus of protein are known to a person skilled in the art. However, a preferred post-translational modification according to the present invention blocking the N-terminus is the pyro-Glu-modification. Pyro-Glu-modification according to the present invention relates to the modification of an N-terminal glutamine by cyclisation of the glutamine via condensation of the α-amino group with a side chain carboxyl group. Modified proteins show an increased half-life. Such a modification can also occur at a glutamate residue.

The antibodies used according to the invention, i.e. antibodies specifically binding CD95L and/or inhibiting CD95L, may be monoclonal, polyclonal or chimeric. However, also single chain antibodies or functional antibody fragments may be used.

For the production of antibodies according to the invention, various hosts including goats, rabbits, rats, mice, humans, and others, may be immunized by injection with the protein or any fragment or oligopeptide thereof which has immunogenic properties. Depending on the host species, various adjuvants may be used to increase immunological response. It is preferred that the peptides, fragments or oligopeptides used to induce antibodies to the protein have an amino acid sequence consisting of at least five amino acids, and more preferably at least 10 amino acids. Monoclonal antibodies to the proteins may be prepared using any technique that provides for the production of antibody molecules by continuous cell lines in culture. These include, but are not limited to, the hybridoma technique, the human B-cell hybridoma technique. In addition, techniques developed for the production of 'chimeric antibodies', the splicing of mouse antibody genes to human antibody genes to obtain a molecule with appropriate antigen specificity and biological activity can be used. The antibodies may be used with or without modification, and may be labeled by joining them, either covalently or non-covalently, with, for example, a reporter molecule.

Various immunoassays may be used for screening to identify antibodies having the desired specificity. Numerous protocols for competitive binding and immunoradiometric assays using either polyclonal or monoclonal antibodies with established specificities are well known in the art. Such immunoassays typically involve the measurement of complex formation between the protein, i.e.CD95L and its specific antibody. A two-site, monoclonal-based immunoassay utilizing monoclonal antibodies reactive to two non-interfering protein epitopes are preferred, but a competitive binding assay may also be employed.

A further aspect of the present invention is a method for the treatment of LGG at risk to undergo malignant progression, comprising
(i) determining the expression level of CD95L in a sample from a patient affected with LGG,
(ii) classifying the LGG as being at risk to undergo malignant progression dependent on the level of CD95L expression, and
(iii) treating the patient.

In step (i) of the method of the invention, the expression of CD95L can be determined by any known method. For example, determination of CD95L can be accomplished as described hereinabove. In a preferred embodiment, the expression of CD95L us determined by contacting the sample with an agent specifically binding to CD95L, preferably an agent as defined hereinabove. According to an especially preferred embodiment, the agent specifically binding to CD95L is also an inhibitor of CD95L.

The LGG is classified as being at risk to undergo malignant progression if the level of CD95L exceeds a predetermined threshold value, preferably as defined herein above.

The treatment of the patient in step (iii) may include any procedure known in the art for the treatment of glioma. In particular, the treatment may include surgery, chemotherapy and/or radiation.

In a particularly preferred embodiment of the invention, the treatment comprises administering an inhibitor of CD95L, in particular an inhibitor as defined hereinabove. The inhibitor may for example comprise
(i) a fusion protein comprising at least one extracellular CD95 domain or a functional fragment thereof and at least one Fc domain or a functional fragment thereof and/or
(ii) an anti-CD95L specific antibody or a CD95L recognizing fragment thereof.

Of course, the inhibitors may be administered in combination with further chemotherapeutic drugs and/or surgery.

The fusion protein according to this embodiment is preferably selected from APG101 as described herein, polypeptides having at least 70% identity to APG101 and functional fragments of APG101.

Another aspect of the present invention is an inhibitor of CD95L for use in the treatment of LGG at risk to undergo malignant progression. The inhibitor of CD95L is as defined hereinabove. Preferably, the inhibitor of CD95L is
(i) a fusion protein comprising at least one extracellular CD95 domain or a functional fragment thereof and at least one Fc domain or a functional fragment thereof and/or
(ii) an anti-CD95L specific antibody or a CD95L recognizing fragment thereof.

The fusion protein according to this embodiment is preferably selected from APG101 as described herein, polypeptides having at least 70% identity to APG101 and functional fragments of APG101.

In the therapeutic uses and methods as described herein, the inhibitor of CD95L is preferably administered at usual dosages that a person skilled in the art is aware of. The period of time in which the inhibitor of CD95L is administered is preferably also the usual period of time for these compounds known to the person skilled in the art. As indicated above, not only the dosage of the administered composition used but also the dosage of the respective active agents, i.e. the inhibitor of CD95L, may vary, depending, for example, on the specific active agents used, the method of administration and the judgment of a prescribing physician. The period of time in which each active agent is administered and the dosage of the active agent may vary, depending on the subject to be treated and on the condition of the subject, e.g. a subject's weight, the subject's age and the type and severity of the disease, in particular cancer, to be treated.

The inhibitor of CD95L may be administered alone or in combination with at least one other agent, such as stabilizing compound, which may be administered in any sterile, biocompatible pharmaceutical carrier, including, but not limited to, saline, buffered saline, dextrose, and water. A pharmaceutical composition comprising the inhibitor of CD95L may be administered to a patient alone or in combination with other agents, drugs or hormones. Any pharmaceutical compositions utilized in this invention may be administered by any number of routes including, but not limited to, oral, intravenous, intramuscular, intra-arterial, intramedullary, intrathecal, intraventricular, transdermal, subcutaneous, intraperitoneal, intranasal, enteral, topical, sublingual or rectal means.

In addition to the active ingredients, pharmaceutical compositions for use according to the invention may contain suitable pharmaceutically-acceptable carriers comprising excipients and auxiliaries, which facilitate processing of the active compounds into preparations, which can be used pharmaceutically. Further details on techniques for formulation and administration may be found in the latest edition of Remington's Pharmaceutical Sciences (Maack Publishing Co., Easton, Pa.).

Pharmaceutical compositions suitable for use in the invention include compositions wherein the active ingredients are contained in an effective amount to achieve the intended purpose. The determination of an effective dose is well within the capability of those skilled in the art. For any compounds, the therapeutically effective dose can be estimated initially either in cell culture assays, e.g., of preadipocyte cell lines or in animal models, usually mice, rabbits, dogs or pigs. The animal model may also be used to determine the appropriate concentration range and route of administration. Such information can then be used to determine useful doses and routes for administration in humans. A therapeutically effective dose refers to that amount of active ingredient, for example a nucleic acid or a protein of the invention or an antibody, which is sufficient for treating a specific condition. Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. Pharmaceutical compositions, which exhibit large therapeutic indices, are preferred. The data obtained from cell culture assays and animal studies are used in formulating a range of dosage for human use. The dosage contained in such compositions is preferably within a range of circulating concentrations that include the ED50 with little or no toxicity. The dosage varies within this range depending upon the dosage from employed, sensitivity of the patient, and the route of administration. The exact dosage will be determined by the practitioner, in light of factors related to the subject that requires treatment. Dosage and administration are adjusted to provide sufficient levels of the active moiety or to maintain the desired effect. Factors, which may be taken into account, include the severity of the disease state, general health of the subject, age, weight, and gender of the subject, diet, time and frequency of administration, drug combination(s), reaction sensitivities, and tolerance/response to therapy.

Long-acting pharmaceutical compositions may be administered every 3 to 4 days, every week or once every two weeks depending on half-life and clearance rate of the particular formulation. Normal dosage amounts may vary from 0.1 to 100,000 micrograms, up to a total dose of about 1 g, depending upon the route of administration. Guidance as to particular dosages and methods of delivery is provided in the literature and generally available to practitioners in the art. Those skilled in the art employ different formulations for nucleotides than for proteins or their inhibitors. Similarly, delivery of polynucleotides or polypeptides will be specific to particular cells, conditions, locations, etc.

### BRIEF DESCRIPTION OF THE DRAWINGS

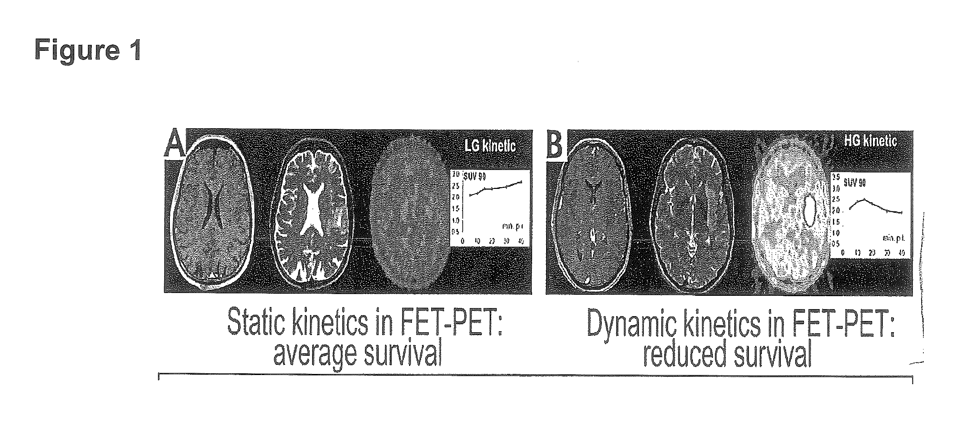

### Figure 1: Dynamic FET-PET measurements can separate LGGs which remain as benign tumors from LGGs which undergo malignant progression.

**(A)** A LGG-patient undergoing molecular imaging (T1 and T2 weighted MRI, left and middle image) and dynamic FET-PET (right image, measurement over 40 min.) and kinetics of the FET-PET signal are recorded (trace); the constant increase in signal strength shows an example for static FET-PET kinetics and indicates a benign glioma without malignant progression.
**(B)** Recordings were performed as in (A); here the dynamic FET-PET kinetics (rise and fall of the signal strength, as indicated by the trace) point out a patient with a LGG that is progressing into a more malignant tumor; modified from Kunz et al. (ref 4)

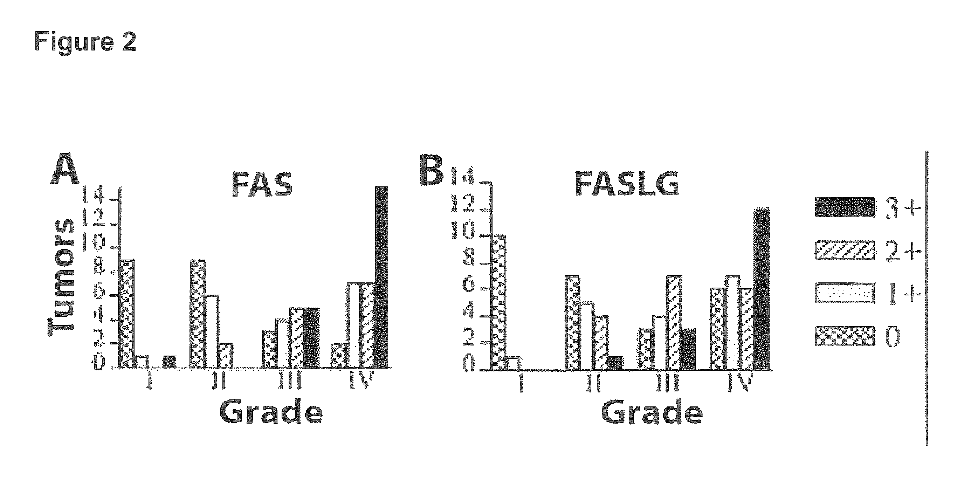

### Figure 2: CD95 and CD95L expression levels in gliomas.

Expression levels for CD95 (A) or CD95L (B) were explored by immunohistochemistry in a range of gliomas (from grade-I to grad-IV); from J. Neuro-Oncol. 1999, 44: 205-12.

### Figure 3: CD95L (FASLG) expression levels separate LGGs that are at risk to undergo malignant progression from benign LGGs.

**(A)** Dynamic FET-PET and histopathological inspection by neuropathologists were used to define a LGG patient-cohort with benign tumors (static FET-PET traces; green) from tumors showing signs for anaplasia (dynamic FET-PET traces; red). The CD95L (FASLG) expression levels in these samples were determined (by immunohistochemistry) and quantified (indicate by colors, light green to purple: weak, up to 10 % of cells in the biopsy are CD95L-positive); moderate: 25 % of cells, strong: up to 50 %; very strong: more than 75 %); note that benign tumors show no or weak CD95L staining while anaplastic LGGs have strong and very strong CD95L expression.
**(B)** H&E stained sample from an anaplastic LGG, the tumor border is outlined by dots.
**(C)** The sample from (B) was CD95L stained and counterstained by hematoxilin, resulting in a brown (DAB) color-precipitate for CD95L and blue nuclei (see insert in B); note that the CD95L expression is largely restricted to the tumor area in the lower part of the image.
**(D+E)** A benign LGG was stained as described in (A) and (B), blood vessels are highlighted by arrows; not the absence of CD95L staining in benign LGG. Scale bars represent: 300 µm in B, C, D and E and 5 µm in the insert of C.

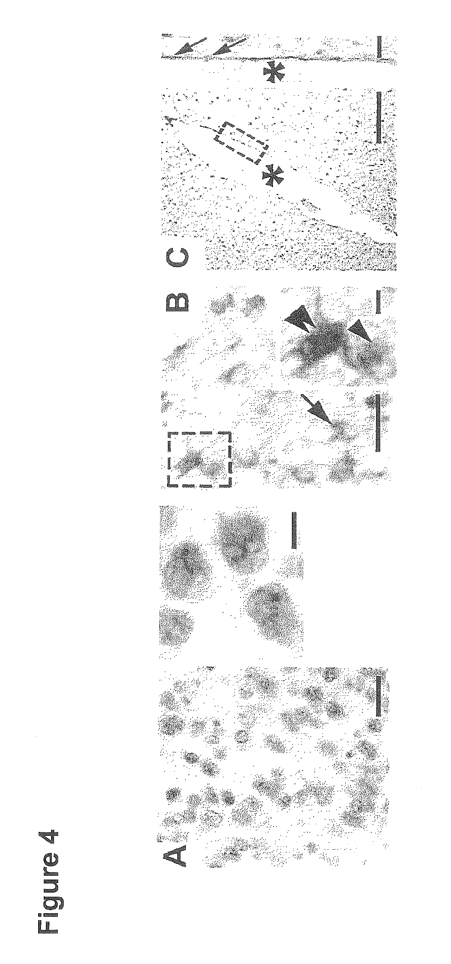

### Figure 4: CD95L (FASLG) in tumor cells of LGG undergoing anaplastic progression.

**(A)** CD95L (FASLG) expression (brown color; DAB) in an anaplastic LGG, counterstained with hematoxilin highlighting nuclei in blue (see also insert in A).
**(B)** Immunohistochemical colabeling for CD95L (blue) and mut-IDH1 (brown); single labeled cells for CD95L in blue (arrow), mut IDH1 in brown (single arrowhead) and double labeled cells in blue and brown (double arrowheads) are indicated; the area encircled by the dashed line was magnified (insert in B).
**(C)** A LGG sample (from a tumor with anaplastic progression) containing a CD95L-positive blood vessel (the vessel-lumen is indicated by an asterisk); the area encircled by the dashed line was magnified (insert in C) showing CD95L expression endothelial cells (arrows). Scale bars represent: 20 µm in A and B, 300 µm in C; 5 µm in the insert of A and B, 20 µm in the insert of C.

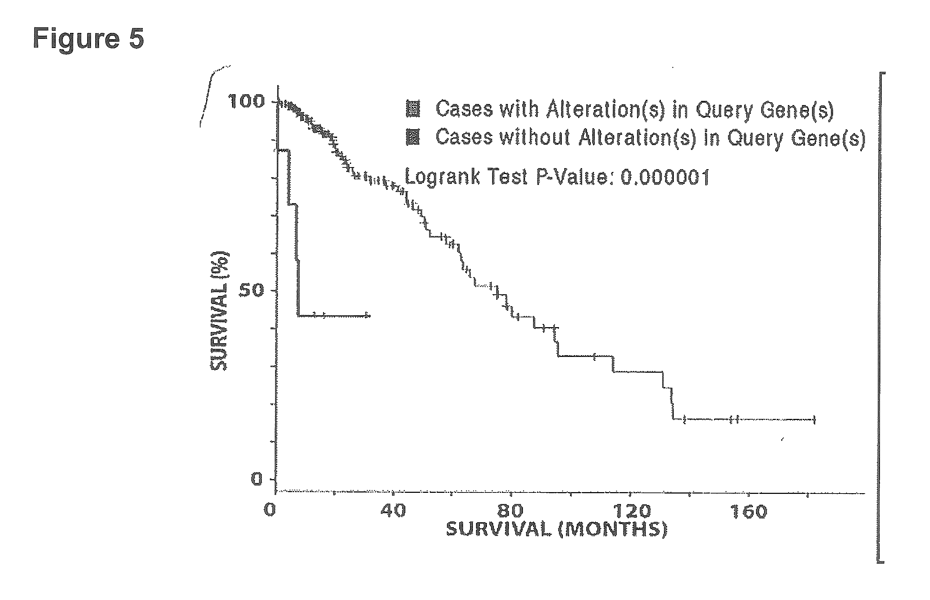

### Figure 5: High CD95L expression levels are associated with decreased survival in LGG patients.

TCGA data (Brain, lower grade glioma, TCGA provisional, all complete tumors) were queried with the mskcc genome browser (https://www.cbioportal.org/public-portal)) and overall survival was correlated with CD95L expression (mRNA expression, RNAseq; z-score ± 1.5); note that high CD95L levels identify a subset of LGG patients with strongly reduced survival probability.

### EXAMPLES

### EXAMPLE 1 - Separation of LGGs with science for anaplastic progression from benign LGGs using FET-PET

The FET-PET molecular imaging approach was used to separate LGGs with science for anaplastic progression (as indicated by FET-PET traces with dynamic kinetics) from benign LGGs (static FET-PET kinetics). Figure 1 shows the results of the dynamic FET-PET measurements. LGG patients were examined using molecular imaging and dynamic FET-PET. The left and middle image in Fig. 1(A) and (B) shows T1 and T2 weighted MRI, while the right image shows the results of dynamic FET-PET measurement over 40 minutes. The kinetics of the FET-PET signal are recorded (trace). In Fig. 1 (A), the constant increase in signal strength shows an example for static FET-PET kinetics and indicates a benign glioma without malignant progression. In Fig. 1(B), the dynamic FET-PET kinetics (rise and fall of the signal strength, as indicated by the trace) point out a patient with an LGG that is progressing into a more malignant tumor.

### EXAMPLE 2 - Correlation of FET-PET results with CD95L expression levels in LGGs

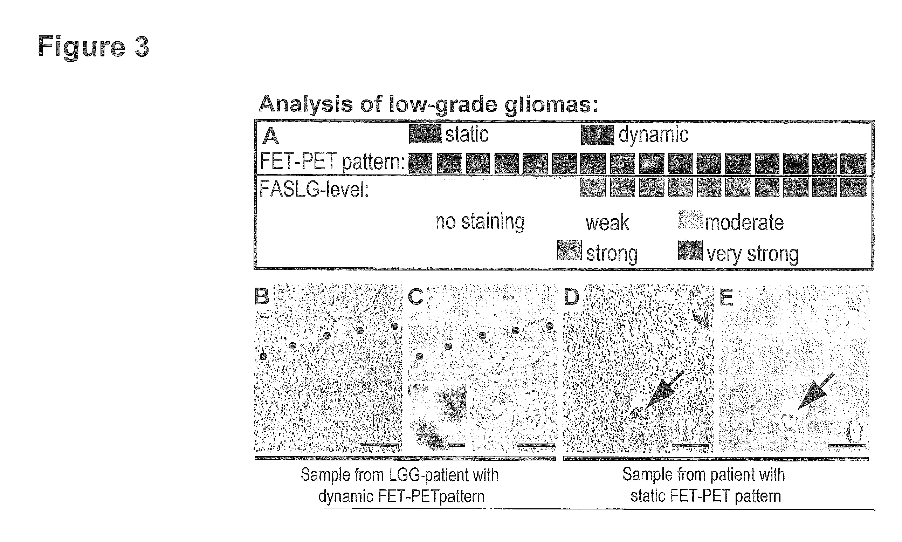

The FET-PET molecular imaging approach to separate LGGs with science for anaplastic progression (as indicated by FET-PET traces with dynamic kinetics) from benign LGGs (static FET-PET kinetics) was correlated with the CD95L levels in both sets of tumor specimen. As shown in Fig. 3, it was found that high levels of CD95L expression are confined to the subset of LGGs which are at risk for malignant progression, while CD95L is absent from benign LGG.

### EXAMPLE 3 - CD95L in tumor cells of LGG undergoing anaplastic progression

The intratumoral cell types that express CD95L in LGG were identified. It was observed that immunohistochemical labeling of CD95L was co-expressed with CD45 only in a smaller subset of intratumoral cells and that very few cells show colabeling for CD95L and active caspase-3 (not shown). However, CD95L was abundant in cells showing specific staining for the tumor cell marker mutant isocitrate-dehydrogenase-1 (mut-IDH1); see Fig. 4. Altogether, this suggests that CD95L is expressed largely in tumor cells of anaplastic LGG and does not mediate intratumoral cells-death. We also noted that CD95L can be expressed in some intra- and peri-tumoral blood vessels in LGG (see Fig. 4C). Overall, our data show that abundant CD95L expression is restricted to LGGs which are at risk for malignant progression (as judged by molecular imaging) and that CD95L is mainly confined to tumor cells and not to tumor parenchyma).

### EXAMPLE 4 - Correlation of CD95L expression levels with survival in LGG patients

To explore if CD94L levels can indeed identify LGGs that transform into higher rate tumors in a larger cohort of individuals, the CD95L expression levels from a TCGA study was compared with overall patient survival. The results are shown in Fig. 5.

### Reference List

All literature cited in this application are incorporated herein by reference.
1. Louis, D.N. et al. The 2007 WHO classification of tumours of the central nervous system. Acta neuropathologica 114, 97-109, (2007).
2. Wessels, P.H. et al. Supratentorial grad II astrocytoma: biological features and clinical course. Lancet neurology 2, 395-403, (2003).
3. Omuro, A. & DeAngelis, L.M. Glioblastoma and other malignant gliomas: a clinical review. JAMA 310, 1842-1850, (2013).
4. Kunz, M. et al. Hot spots in dynamic (18) FET-PET delineate malignant tumor parts within suspected WHO grade II gliomas. Neuro-oncology 13, 307-316, (2011).
5. Niyazi, M. et al. FET-PET for malignant glioma treatment planning. Radiother Oncol 99, 44-48, (2011).
6. Frankel, B., Long, S.L. & Ryken, T.C. Human astrocytomas co-expressing Fas and Fas ligand also produce TGFbeta2 and Bcl-2. Journal of neuro-oncology 44, 205-212, (1999).

## Claims

1. A method for the diagnosis of low grade glioma (LGG) at risk to undergo malignant progression, comprising
(i) determining the expression level of CD95L in a sample from a patient affected with LGG and
(ii) classifying the LGG as being at risk to undergo malignant progression dependant on the level of CD95L expression.

2. A method for the prognosis of malignant progression of LGG, comprising
(i) determining the expression level of CD95L in a sample from a patient affected with LGG and
(ii) classifying the LGG as being at risk to undergo malignant progression dependant on the level of CD95L expression.

3. The method of claim 1 or 2, wherein the expression of CD95L in the sample is determined by contacting the sample with an agent specifically binding to CD95L.

4. The method of any one of the preceding claims, wherein the agent specifically binding to CD95L comprises
(i) a fusion protein comprising at least one extracellular CD95 domain or a functional fragment thereof and at least one Fc domain or a functional fragment thereof and/or
(ii) an anti-CD95L specific antibody or a CD95L recognising fragment thereof.

5. The method of claim 4, wherein the fusion protein is selected from APG101, polypeptides having at least 70% identity to APG101 and functional fragments of APG101.

6. The method of any of the preceding claims, wherein the low grade glioma is a WHO Grade I or II glioma.

7. The method of claim 6, wherein the low grade glioma is a WHO Grade II glioma.

8. A method for treating a patient affected with LGG at risk to undergo malignant progression, comprising
(i) determining the expression level of CD95L in a sample from said patient,
(ii) classifying the LGG as being at risk to undergo malignant progression dependent on the level of CD95L expression, and
(iii) treating the patient.

9. The method of claim 8, wherein in step (i) the expression of CD95L in the sample is determined by contacting the sample with an agent specifically binding to CD95L, preferably an agent as defined in claim 4 or 5.

10. The method of claim 8 or 9, wherein step (iii) comprises administering to the patient an inhibitor of CD95L, preferably an agent as defined in claim 4 or 5.

11. The method of any one of claims 8-10, wherein the LGG is a WHO Grade I or II glioma.

12. The method of claim 11, wherein the LGG is a WHO Grade II glioma.

13. An inhibitor of CD95L for use in the treatment of LGG at risk to undergo malignant progression.

14. The inhibitor of CD95L for the use of claim 13, wherein the inhibitor is an agent as defined in claim 4 or 5.

15. The inhibitor of CD95L for the use of claim 13 or 14, wherein the LGG is a WHO Grade I or II glioma, in particular WHO Grade II glioma.


SEQUENCE LISTING

<110>  Deutsches Krebsforschungszentrum
       Ludwig-Maximilians-Universität München

<120>  Low grade gliomas (LGG)

<130>  59583P EP

<160>  1

<170>  PatentIn version 3.5

<210>  1
<211>  400
<212>  PRT
<213>  Artificial Sequence

<220>
<223>  Recombinant fusion protein consisting of human CD95 extracellular
       domain with human IgG1 FC-part to its C-terminus



<220>
<221>  SIGNAL
<222>  (1)..(16)
<223>  Variable cleavage sites

<220>
<221>  SIGNAL
<222>  (1)..(20)
<223>  Variable cleavage sites

<220>
<221>  SIGNAL
<222>  (1)..(25)
<223>  Variable cleavage sites

<220>
<221>  DOMAIN
<222>  (26)..(172)
<223>  Human CD95 extracellular domain

<220>
<221>  MOD_RES
<222>  (26)..(26)
<223>  PYRROLIDONE CARBOXYLIC ACID

<220>
<221>  DISULFID
<222>  (59)..(73)

<220>
<221>  DISULFID
<222>  (63)..(82)

<220>
<221>  DISULFID
<222>  (85)..(101)

<220>
<221>  DISULFID
<222>  (104)..(119)

<220>
<221>  DISULFID
<222>  (107)..(127)

<220>
<221>  CARBOHYD
<222>  (118)..(118)
<223>  N-linked Glycosylation at Position N118

<220>
<221>  DISULFID
<222>  (129)..(143)

<220>
<221>  DISULFID
<222>  (135)..(140)

<220>
<221>  CARBOHYD
<222>  (136)..(136)
<223>  N-linked Glycosylation at Position N136

<220>
<221>  DISULFID
<222>  (146)..(157)

<220>
<221>  DISULFID
<222>  (149)..(165)

<220>
<221>  DOMAIN
<222>  (172)..(400)
<223>  Human IgG1-FC domain (one amino acid overlap with CD95 domain)

<220>
<221>  DISULFID
<222>  (173)..(173)
<223>  Interchain cystine forming residue.

<220>
<221>  DISULFID
<222>  (179)..(179)
<223>  Interchain cystine forming residue.

<220>
<221>  DISULFID
<222>  (182)..(182)
<223>  Interchain cystine forming residue

<220>
<221>  DISULFID
<222>  (214)..(274)

<220>
<221>  CARBOHYD
<222>  (250)..(250)
<223>  N-linked Glycosylation at Position N250

<220>
<221>  DISULFID
<222>  (320)..(378)

<400>  1

Met Val Gly Ile Trp Thr Leu Leu Pro Leu Val Leu Thr Ser Val Ala
1               5                   10                  15

Arg Leu Ser Ser Lys Ser Val Asn Ala Gln Val Thr Asp Ile Asn Ser
            20                  25                  30

Lys Gly Leu Glu Leu Arg Lys Thr Val Thr Thr Val Glu Thr Gln Asn
        35                  40                  45

Leu Glu Gly Leu His His Asp Gly Gln Phe Cys His Lys Pro Cys Pro
    50                  55                  60

Pro Gly Glu Arg Lys Ala Arg Asp Cys Thr Val Asn Gly Asp Glu Pro
65                  70                  75                  80

Asp Cys Val Pro Cys Gln Glu Gly Lys Glu Tyr Thr Asp Lys Ala His
                85                  90                  95

Phe Ser Ser Lys Cys Arg Arg Cys Arg Leu Cys Asp Glu Gly His Gly
            100                 105                 110

Leu Glu Val Glu Ile Asn Cys Thr Arg Thr Gln Asn Thr Lys Cys Arg
        115                 120                 125

Cys Lys Pro Asn Phe Phe Cys Asn Ser Thr Val Cys Glu His Cys Asp
    130                 135                 140

Pro Cys Thr Lys Cys Glu His Gly Ile Ile Lys Glu Cys Thr Leu Thr
145                 150                 155                 160

Ser Asn Thr Lys Cys Lys Glu Glu Gly Ser Arg Ser Cys Asp Lys Thr
                165                 170                 175

His Thr Cys Pro Pro Cys Pro Ala Pro Glu Leu Leu Gly Gly Pro Ser
            180                 185                 190

Val Phe Leu Phe Pro Pro Lys Pro Lys Asp Thr Leu Met Ile Ser Arg
        195                 200                 205

Thr Pro Glu Val Thr Cys Val Val Val Asp Val Ser His Glu Asp Pro
    210                 215                 220

Glu Val Lys Phe Asn Trp Tyr Val Asp Gly Val Glu Val His Asn Ala
225                 230                 235                 240

Lys Thr Lys Pro Arg Glu Glu Gln Tyr Asn Ser Thr Tyr Arg Val Val
                245                 250                 255

Ser Val Leu Thr Val Leu His Gln Asp Trp Leu Asn Gly Lys Glu Tyr
            260                 265                 270

Lys Cys Lys Val Ser Asn Lys Ala Leu Pro Ala Pro Ile Glu Lys Thr
        275                 280                 285

Ile Ser Lys Ala Lys Gly Gln Pro Arg Glu Pro Gln Val Tyr Thr Leu
    290                 295                 300

Pro Pro Ser Arg Glu Glu Met Thr Lys Asn Gln Val Ser Leu Thr Cys
305                 310                 315                 320

Leu Val Lys Gly Phe Tyr Pro Ser Asp Ile Ala Val Glu Trp Glu Ser
                325                 330                 335

Asn Gly Gln Pro Glu Asn Asn Tyr Lys Thr Thr Pro Pro Val Leu Asp
            340                 345                 350

Ser Asp Gly Ser Phe Phe Leu Tyr Ser Lys Leu Thr Val Asp Lys Ser
        355                 360                 365

Arg Trp Gln Gln Gly Asn Val Phe Ser Cys Ser Val Met His Glu Ala
    370                 375                 380

Leu His Asn His Tyr Thr Gln Lys Ser Leu Ser Leu Ser Pro Gly Lys
385                 390                 395                 400